# EUROPEAN PATENT SPECIFICATION

(11) **EP 4 099 925 B1**
(45) Date of publication and mention of the grant of the patent: **19.07.2023**
(21) Application number: 21704456.9
(22) Date of filing: 04.02.2021
(51) Int. Cl.: A61B 17/17, A61B 17/16, A61B 17/70, A61B 90/00

(54) **UNIVERSAL DRILL GUIDE SYSTEM**
UNIVERSALBOHRFÜHRUNGSSYSTEM
SYSTÈME DE GUIDAGE DE FORAGE UNIVERSEL

(30) Priority: 06.02.2020 US 202062970850 P
(43) Date of publication of application: 14.12.2022
(73) Proprietor: Aesculap AG, 78532 Tuttlingen (DE)
(72) Inventor: SCHWARTZ, Christopher, Middlebury, CT 06762 (US); CORRAO, Ernie, Bethel, CT 06801 (US); CHEN, Kehui, Seymour, CT 06483 (US); SUCHOMEL, Petr, 46001 Liberec (CZ); KOTHE, Ralph, 22397 Hamburg (DE); LITKE, Ronald, Sandy Hook, CT 06482 (US); LARSEN, Scott, Newtown, CT 06470 (US); SROKA, Nicole, Seymour, CT 06483 (US); LEDER, Tamara, 78570 Mühlheim (DE)
(74) Representative: Winter, Brandl - Partnerschaft mbB
(86) International application number: PCT/EP2021/052719
(87) International publication number: WO 2021/156395

(56) References cited:
- WO-A2-2019/118753
- US-A1- 2014 276 880
- US-A1- 2014 343 553

## Description

### TECHNICAL FIELD

The present disclosure relates generally to a universal drill guide system for spinal fixation.

### BACKGROUND

Spinal fusion is a procedure in which damaged vertebrae are removed, and vertebrae adjacent to the removed vertebrae are fused together with graft material. The spine must be immobilized during fusion. To immobilize the spine, one or more fixation rods are anchored to the vertebrae to limit movement.

Fixation rods are anchored to vertebrae using bone screws that are driven into the vertebrae. Before a bone screw is driven into a vertebral body, a hole is prepared in the vertebral body in a minimally invasive manner with help of a universal drill guide system. In one possible procedure, a drill tube is passed through a small incision and navigated to a desired entry point on the vertebral body. A cortical punch is then advanced through the tube to punch a start hole in the cortical layer of bone. The cortical punch is then removed from the tube, and a drill bit is advanced through the tube to the start hole. Once the drill bit is aligned with the start hole, the surgical drill is powered on to drill a hole of a desired diameter and depth. After the screw hole is drilled, the drill guide is removed, and a bone screw can be driven into the screw hole.

When drilling a screw hole, the depth of drilling must be carefully controlled to ensure that the drill bit does not penetrate too far into the vertebral body. One option for control drill depth is to attach some type of drill stop on the drill tube to limit how far the tip of the drill bit extends past the end of drill tube in the patient. This option may be difficult to implement, however, if there are multiple drill bits and drill tubes of different sizes that must be accommodated.

Guiding drill bits to the proper location can be difficult. Therefore, some drill bits have passages that allow the drill bits to be passed over a Kirschner wire or "K-wire". A K-wire can be attached to a bone at a desired location to precisely navigate the drill bit and other instruments to that location. Although K-wires are helpful for navigation, they present a number of challenges when other instruments are used around them.

After drilling is completed, it is sometimes necessary to remove the drill guide from the operating side while leaving the drill tube and the drill bit in place. This can be a technical challenge, because the drill stop is engaged with the drill bit to limit the proximal movement of the drill bit. In the conventional solution, the drill stop is detached/ released from the drill guide in a separate process, leading to a complicated removing process and thus an increased operation duration.

Further prior art is known from US 2014/7343553 A1, which defines the preamble of claim 1.

### SUMMARY

In connection with this, the present disclosure is based on the object of providing a universal drill guide system in which removing of the universal drill guide can be facilitated to shorten the operation duration.

This object is achieved according to the present disclosure a universal drill guide system comprising a universal drill guide, a drill tube insertable into the universal drill guide and a drill bit insertable into the drill tube. The universal drill guide comprises a drill stop (stopper preferable in the form of an abutment) configured to abut on a stop/abutment surface of the drill bit to limit a drilling depth of the drill bit, a locking ring rotatable between a locking orientation, in which the drill tube inserted into the universal drill guide is locked by the locking ring, and a release orientation, in which the drill tube is releasable from the locking ring, and a camming mechanism configured to pivot the drill stop away from the stop surface of the drill bit as the locking ring is rotated into the release orientation. Thereby, when the locking ring of the universal drill guide is rotated into the release orientation and the universal drill guide is released from the drill tube and the drill bit in the proximal direction, the drill stop will be automatically pivoted from the drill bit. In other words, the limitation of the drill bit in the proximal direction by the drill stop is automatically canceled, when the locking ring is in the release orientation. Thus, an additional releasing process of the drill stop from the drill bit is spared and removing of the drill guide is facilitated. Accordingly, the operation duration is shortened.

Advantageous embodiments of the present disclosure are claimed in the subclaims and explained more precisely in the following.

The camming mechanism includes a cam slot formed on the locking ring and a cam following pin formed on a distal end of a shaft of the drill stop.

The cam slot and the cam following pin are configured such that a rotation of the locking ring clockwise causes the drill stop to simultaneously rotate counterclockwise, pivoting the drill stop away from the stop surface of the drill bit. Thereby, a simultaneous rotation of the drill stop and the locking ring can be easily achieved. The releasing process of the universal drill guide can be facilitated.

Further, a compression spring exerts a biasing force on the locking ring to bias the locking ring in the locking orientation.

Preferably, a switch is manually operable to rotate the locking ring from the locking orientation to the release orientation against the biasing force of the compression spring.

The drill stop comprises a stop plate extending from the shaft of the drill stop transversely. The stop plate comprises a slot having a slot dimension to permit a distal section of the drill bit to pass through the stop plate, until the stop surface of the drill bit abuts on the stop plate. In this way, a limitation of the drilling depth is achieved.

The universal drill guide comprises a guide body. The shaft of the drill stop is configured to be raised or lowered with respect to the guide body to set a desired drilling depth. Thus, different drilling depths can be easily selected.

Moreover, the universal drill guide comprises a lock, in particular a spring loaded lock, which is configured to engage with the shaft of the drill stop to lock the shaft with respect to the guide body by exerting a biasing force. Thus, the drill stop will be hold in place with respect to the guide body to limit the desired drilling depth.

The shaft of the drill stop includes further circumferential grooves to engage with the lock.

Furthermore, the lock includes a release button configured to be pressed inwardly to disengage the lock from the shaft of the drill stop. Thereby, the drill depths can be selected by raising or lowering the drill stop relative to the drill body. After selecting the desired drill depth, the release button is released such that the lock engages with the shaft of the drill stop again to hold the drill stop in place.

Preferably, the universal drill guide includes a first set of indicia and a second set of indicia for indicating a selected depth.

More preferably, the first set of indicia includes a vertical series of markings on the guide body and the second set of indicia includes a vertical series of markings on the shaft of the drill stop. Thus, two sets of indicia are provided on two sides of the universal drill guide in the universal drill guide system. This configuration of redundant markings on the two different sides addresses situations where the surgeon can only see one side of the universal drill guide. Accordingly, the usability and the ergonomics can be improved.

### BRIEF DESCRIPTION OF THE FIGURES

The foregoing summary and the following detailed description will be better understood in conjunction with non-limiting examples shown in the drawing figures. Figures 1-15 show a drill guide system according to the claimed invention while figures 16-53 do not show the claimed invention.
FIG. 1 is a perspective view of a universal drill guide system according to one aspect of the present disclosure;
FIG. 2 is a front view of a set of drill tubes for use with the universal drill guide system of FIG. 1;
FIG. 3 is a front view of a set of drill bits for use with the universal drill guide system of FIG. 1;
FIG. 4 is a truncated perspective view of the universal drill guide system of FIG. 1;
FIG. 5 is a truncated side view of the universal drill guide system of FIG. 1, showing a spring loaded lock in a first state;
FIG. 6 is a truncated side view of the universal drill guide system of FIG. 1, showing the spring loaded lock in a second state;
FIG. 7 is a truncated perspective view of a drill tube capture of the universal drill guide system of FIG. 1, showing the drill tube capture in a first position;
FIG. 8 is a truncated perspective view of the drill tube capture of the universal drill guide system of FIG. 1, showing the drill tube capture in a second position;
FIG. 9A is a truncated perspective view of the drill tube capture of the universal drill guide system of FIG. 1 during a first step of inserting a drill tube;
FIG. 9B is a truncated perspective view of the drill tube capture of the universal drill guide system of FIG. 1 during a second step of inserting a drill tube;
FIG. 9C is a truncated perspective view of the drill tube capture of the universal drill guide system of FIG. 1 during a third step of inserting a drill tube;
FIG. 9D is a truncated perspective view of the drill tube capture of the universal drill guide system of FIG. 1 after a final step of inserting a drill tube;
FIG. 10 is an enlarged truncated perspective view of the drill tube capture of the universal drill guide system of FIG. 1 in a locking orientation;
FIG. 11 is an enlarged truncated perspective view of the drill tube capture of the universal drill guide system of FIG. 1 in a release orientation;
FIG. 12 is an enlarged perspective view of components of the universal drill guide system of FIG. 1, showing the components when the drill stop is in a position for engagement with a drill bit;
FIG. 13 is an enlarged perspective view of components of the universal drill guide system of FIG. 1, showing the components when the drill stop is in a position released from a drill bit;
FIG. 14 is an truncated perspective view of the universal drill guide system of FIG. 1, showing the universal drill guide system in a condition to be detached from a drill tube;
FIG. 15 is another truncated perspective view of the universal drill guide system of FIG. 1, showing the universal drill guide system being detached from the drill tube;
FIG. 16 is a perspective view of a cannulated drill bit and a K-wire according to another aspect of the present disclosure;
FIG. 17 is an enlarged truncated view of the cannulated drill bit, the K-wire and a K-wire retention mechanism according to another aspect of the present disclosure;
FIG. 18 is an enlarged truncated cross section view of the cannulated drill bit, the K-wire, and the K-wire retention mechanism of FIG. 17;
FIG. 19 is an enlarged perspective view of a K-wire according to another aspect of the present disclosure, showing a midsection of the K-wire;
FIG. 20 is an enlarged perspective view of a K-wire retention mechanism according to another aspect of the present disclosure;
FIG. 21 is a truncated perspective view of the cannulated drill bit, the K-wire and the K-wire retention mechanism according to another aspect of the present disclosure, with a force retention sleeve shown in a first position;
FIG. 22 is a truncated perspective view of the cannulated drill bit, the K-wire and the K-wire retention mechanism of FIG. 21, with the force retention sleeve shown in a second position;
FIG. 23 is an enlarged truncated perspective view of a cannulated drill bit, a K-wire and a K-wire retention mechanism according to another aspect of the present disclosure, with the cannulated drill bit attached to a drill driver;
FIG. 24 is another enlarged truncated perspective view of the cannulated drill bit, the K-wire and the K-wire retention mechanism of FIG. 23, with some features shown transparent for clarity;
FIG. 25 is a perspective view of a drill removal tool according to another aspect of the present disclosure;
FIG. 26 is a truncated perspective view of the drill removal tool of FIG. 25 during attachment to a cannulated drill bit and a K-wire;
FIG. 27 is a truncated side view of a wire clamp according to another aspect of the present disclosure, showing the wire clamp in an unclamped state;
FIG. 28 is a truncated side view of the wire clamp of FIG. 27, showing the wire clamp in a clamped state;
FIG. 29 is an enlarged truncated cross section view of the wire clamp of FIG. 27;
FIG. 30 is a truncated perspective view of components of the drill removal tool of FIG. 25;
FIG. 31 is another truncated perspective view of components of the drill removal tool of FIG. 25;
FIG. 32 is another truncated perspective view of the drill removal tool of FIG. 25, shown during removal of the drill bit from the patient;
FIG. 33 is a perspective view of a bone screw driver with a K-wire retention module according to another aspect of the present disclosure, with the bone screw driver and the K-wire retention module passed over a K-wire;
FIG. 34 is an enlarged truncated side view of a distal end of the bone screw driver of FIG. 33;
FIG. 35 is an enlarged truncated perspective view of the distal end of the bone screw driver of FIG. 33;
FIG. 36 is another perspective view of the bone screw driver with the K-wire retention module of FIG. 33 during advancement over a K-wire;
FIG. 37 is an enlarged truncated perspective view of the bone screw driver with the K-wire retention module of FIG. 33, showing internal components of the K-wire retention module;
FIG. 38 is a side view of the K-wire retention module of FIG. 33, with some components removed for clarity, the K-wire retention module shown in a first mode of operation;
FIG. 39 is an enlarged truncated perspective view of the K-wire retention module components of FIG. 38 in the first mode of operation;
FIG. 40 is a side view of the K-wire retention module of FIG. 33, with some components removed for clarity, the K-wire retention module shown in a second mode of operation;
FIG. 41 is an enlarged truncated perspective view of the K-wire retention module components of FIG. 40 in the second mode of operation;
FIG. 42 is a perspective view of an alternate bone screw driver with a K-wire retention module according to another aspect of the present disclosure, with the bone screw driver and the K-wire retention module passed over a K-wire;
FIG. 43 is a perspective view of the bone screw driver with the K-wire retention module of FIG. 42, showing internal components of the K-wire retention module in a first mode of operation;
FIG. 44 is an enlarged perspective view of the internal components of the K-wire retention module of FIG. 43 in the first mode of operation;
FIG. 45 is an enlarged perspective view of the internal components of the K-wire retention module of FIG. 43 in a second mode of operation;
FIG. 46 is an enlarged truncated perspective view of the drill removal tool of FIG. 25, showing one step of an alternate technique for using the drill removal tool;
FIG. 47 is another enlarged truncated perspective view of the drill removal tool of FIG. 25, showing another step of the alternate technique;
FIG. 48 is another truncated perspective view of the drill removal tool of FIG. 25, showing another step of the alternate technique;
FIG. 49 is another truncated perspective view of the drill removal tool of FIG. 25, showing another step of the alternate technique;
FIG. 50 is another enlarged truncated side view of the drill removal tool of FIG. 25, showing another step of the alternate technique;
FIG. 51 is another enlarged truncated side view of the drill removal tool of FIG. 25, showing another step of the alternate technique;
FIG. 52 is another truncated perspective view of the drill removal tool of FIG. 25, showing another step of the alternate technique; and
FIG. 53 is another truncated perspective view of the drill removal tool of FIG. 25, showing another step of the alternate technique.

### DETAILED DESCRIPTION

The following section describes different instruments used for fixation of the cervical spine according to the present disclosure.

In the present disclosure, "distal" basically means "in a direction away from a user/ surgeon towards a patient" and "proximal" basically means "in a direction towards the user/ surgeon away from the patient".

### Universal Drill Guide System

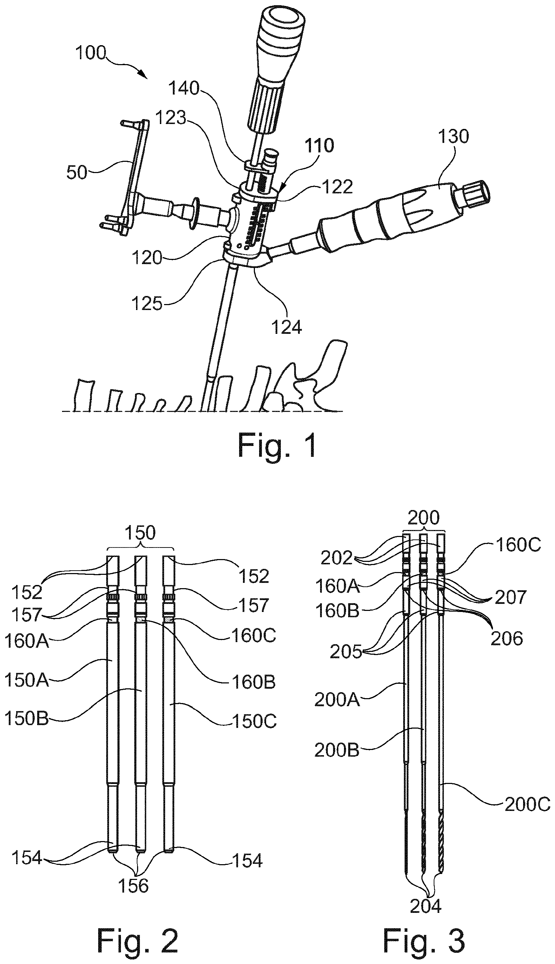

Referring to FIGS. 1-3, a universal drill guide system 100 is shown according to one example. The universal drill guide system 100 features a universal drill guide 110. The universal drill guide 110 includes a tubular guide body 120 and a handle 130 that extends obliquely from the guide body 120. The guide body 120 has a proximal end 122 defining a proximal opening 123 for receiving a drill bit, as will be described. The guide body 120 also has a distal end 124 defining a distal opening 125 for receiving a drill tube, as will be described. Furthermore, the guide body 120 features a drill stop 140 that limits drilling depth by limiting how far a drill bit is advanced through the guide body 120. An optional navigation star unit 50 is attached to the universal drill guide 110 in FIG. 1, which can be calibrated and used with conventional navigation systems.

The universal drill guide system 100 also includes a set of interchangeable drill tubes 150 and a set of interchangeable drill bits 200. The set of interchangeable drill tubes 150 includes a first drill tube 150A, a second drill tube 150B, and a third drill tube 150C. The set of interchangeable drill bits 200 includes a first drill bit 200A, a second drill bit 200B and a third drill bit 200C. The number of the drill tubes 150 and the number of drill bits 200 are however not restricted to three. All of the drill tubes 150A, 150B and 150C, and all of the drill bits 200A, 200B and 200C are connectable to the universal drill guide 110.

The first, second and third drill bits 200A, 200B and 200C are cannulated and have features designed to retain a K-wire, as will be explained in the next section of this description. The first, second and third drill bits 200A, 200B and 200C also have different drilling diameters. Each drilling diameter is configured to drill a screw hole of a specific size into a vertebral body. The first, second and third drill bits 200A, 200B and 200C each have a proximal end 202 for attachment to a drill driver and an opposite distal end 204 with cutting edges to drill a hole. The first, second and third drill bits 200A, 200B and 200C also have a reduced diameter section 205 adjacent to an enlarged diameter section 207, forming an abrupt transition. This abrupt transition forms a stop surface 206 located between the proximal end 202 and the distal end 204. The stop surfaces 206 cooperate with the drill stop 140 on the guide body 120 to limit how far the drill bits can be advanced into the vertebral body during drilling. The stop surfaces 206 also play a role in removing the drill bits 200A, 200B and 200C after a drilling operation, as will be explained in another section.

The first, second and third drill tubes 150A, 150B and 150C are designed to guide the advancement of the first, second and third drill bits 200A, 200B and 200C, respectively, during drilling. The first, second and third drill tubes 150A, 150B and 150C also keep the first, second and third drill bits 200A, 200B and 200C axially stable during drilling. Each of the first, second and third drill tubes 150A, 150B and 150C has a proximal end 152 for attachment to the guide body 120 and an opposite distal end 154 that is inserted into the patient at the drilling location. The proximal end 152 of each of the drill tubes 150A, 150B and 150C includes a notch 157 that facilitates attachment to the guide body 120, as will be described in more detail.

The first, second and third drill tubes 150A, 150B and 150C define a drill bit passages 156 having inner diameters specially sized to receive the first, second and third drill bits 200A, 200B and 200C, respectively. Thus, the first, second and third drill bits 200A, 200B and 200C are designed to only work with the first, second and third drill tubes 150A, 150B and 150C, respectively. Indicia are provided on each of the first, second and third drill tubes 150A, 150B and 150C, and each of the first, second and third drill bits 200A, 200B and 200C, to assist the user in selecting the proper drill tube for the selected drill bit. Any type of indicia can be used. In the system 100, the first drill tube 150A and the first drill bit 200A have matching indicia 160A, the second drill tube 150B and the second drill bit 200B have matching indicia 160B, and the third drill tube 150C and the third drill bit 200C have matching indicia 160C. The indicia 160A, 160B and 160C are unique and different from one another, and appear in the form of different colored bands around the circumference of each drill tube and drill bit.

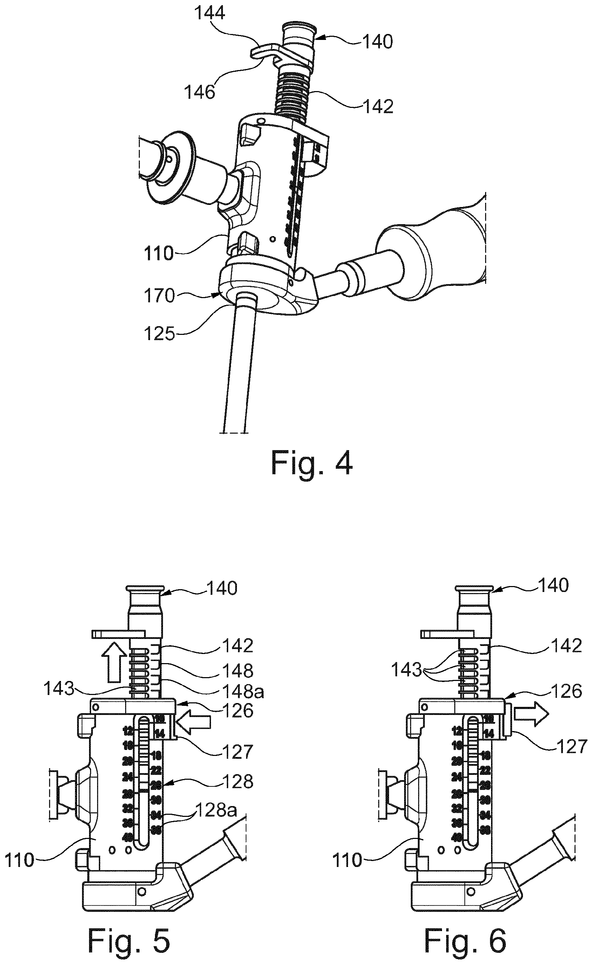

Referring to FIG. 4, the drill stop 140 has a shaft 142 and a stop plate 144 extending laterally from the shaft 142. The stop plate 144 has a slot 146 having a slot dimension that is wide enough to permit passage of the distal sections of the first, second and third drill bits 200A, 200B and 200C through the stop plate 144. The slot 146 is smaller than the cross sectional dimension of the stop surfaces 206 of the first, second and third drill bits 200A, 200B and 200C. In this arrangement, the stop plate 144 is configured to permit advancement of each of the first, second and third drill bits 200A, 200B and 200C through the stop plate 144 up until their respective stop surfaces 206 abut on the stop plate 144. At such time, the drill bit has reached the selected drill depth, and further advancement of the drill bit through the guide body 120 is prevented.

Referring to FIGS. 5 and 6, the drill stop 140 can be raised or lowered to set a desired drilling depth setting. A spring loaded lock 126 releasably engages with the shaft 142 of the drill stop 140 to lock and unlock the shaft 142. The shaft 142 has a series of circumferential grooves 143 in one side. The spring loaded lock 126 is configured to engage with one of the grooves 143 to lock the position of the shaft 142 under a spring bias. The spring loaded lock 126 includes a release button 127 that can be pressed inwardly as shown in FIG. 5. Pressing the release button 127 inwardly disengages the spring loaded lock 126 from the shaft 142 so the shaft 142 can be raised or lowered relative to the guide body 120 to set the depth setting. Once the depth setting is set, the release button 127 is released as shown in FIG. 6 to allow the spring loaded lock 126 to engage with the shaft 142 under the spring bias. Engagement of the spring loaded lock 126 with the shaft 142 locks the vertical position of the shaft 142 relative to the guide body 120 and fixes the depth setting.

The universal drill guide 110 includes two sets of indicia on the exterior that provide the user with a visual indicator of the selected depth setting. A first set of indicia 128 include a vertical series of markings 128a on the side of the guide body 120. A second set of indicia 148 include a vertical series of markings 148a on the shaft 142. Each of the marking 128a, 148a is labeled with a unique number corresponding to a depth in millimeters or other unit of measure. The markings 128a are oriented on a different side of the universal drill guide 110 than the markings 148a. This placement of redundant markings on two different sides addresses situations where the surgeon can only see one side of the universal drill guide 110.

The guide body 120 includes an auto drill tube capture 170 that allows each of the first, second and third drill tubes 150A, 150B and 150C to be connected to the distal opening 125 in a quick-connect coupling/ with a quick-fit connection. The auto drill tube capture 170 allows a user to insert one of the drill tubes 150A, 150B and 150C into the guide body 120 and lock it in place without touching any locking mechanisms on the guide body 120. The drill tube is simply inserted into the distal opening 125 until it engages with an automatic lock. Each of the drill tubes 150A, 150B and 150C has a different inner diameter to accommodate one of the drill bits, as noted above. However, all of the drill tubes 150A, 150B and 150C have the same outer dimensions that engage with the drill stop 140 and the auto drill tube capture 170. Therefore, all of the drill tubes 150A, 150B and 150C interact with the auto drill tube capture 170 the same way.

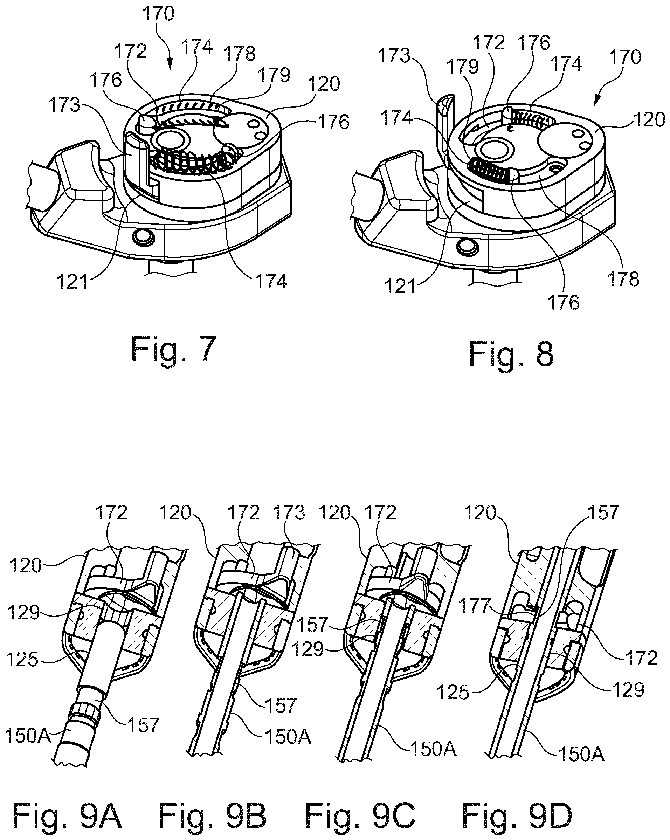

Referring to FIGS. 7 and 8, the auto drill tube capture 170 includes a spring loaded locking ring 172 inside the guide body 120. The locking ring 172 is configured to snap into the notches 157 formed in each of the drill tubes 150A, 150B and 150C. The locking ring 172 is rotatable in the guide body 120 between a locking orientation and a release orientation. A pair of compression springs 174 exert a counterclockwise biasing force on the locking ring 172 to bias the locking ring 172 in the locking orientation. Each compression spring 174 bears against a locking tab 176 that extends in a proximal direction or upwardly from the rest of the locking ring 172.

The locking tabs 176 and the compression springs 174 are captive in a pair of slots 178. One end of each compression spring 174 bears against an end wall 179 of the slot 178, and the opposite end of the spring 174 bears against the locking tab 176. In this arrangement, the locking ring 172 is maintained in the locking orientation unless the user manually rotates the locking ring 172 to the release orientation. The locking ring 172 can be rotated by exerting a clockwise force on a switch 173 that is attached to the locking ring. The switch 173 extends through an elongated aperture 121 in the guide body 120 and is exposed on the exterior of the guide body 120.

When the locking ring 172 is in the locking orientation, as shown in FIG. 7, the compression springs 174 have released energy to push the locking tabs 176 in a counterclockwise direction in their respective slots 178 to rotate the locking ring 172 counterclockwise. When the locking ring 172 is in the release orientation, as shown in FIG. 8, the locking tabs 176 are rotated in a clockwise direction to compress the compression springs 174 in their respective slots 178 under stored energy. The user can move the locking ring 172 from the locking orientation to the release orientation by manually rotating the switch 173 in the clockwise direction.

FIGS. 9A-9D show a sequence in which a drill tube, in this example the first drill tube 150A, is inserted into the guide body 120 and locked in place by the locking ring 172. The same sequence occurs when the second drill tube 150B and the third drill tube 150C are inserted into the guide body 120.

The drill tube 150A is inserted through the distal opening 125 and into a passage 129 formed in the guide body 120, as shown in FIG. 9A. The locking ring 172 is initially disposed in the locking orientation. The drill tube 150A is advanced into the passage 129 until it enters the locking ring 172, as shown in FIG. 9B. As the drill tube 150A continues to advance, the drill tube 150A contacts an interior surface of the locking ring 172, as shown in FIG. 9C. This contact between the drill tube 150A and the locking ring 172 causes the locking ring 172 to rotate out of the locking orientation. Contact with the locking ring 172 occurs at a chamfer 177 on the locking ring 172 that extends into the passage 129. The proximal end and the sidewall of the drill tube 150A contact the chamfer 177 as the drill tube 150A is advanced proximally in the passage 129. The orientation of the chamfer 177 is configured such that the side wall of the drill tube 150A deflects the locking ring 172 a small distance or a small anglein a counterclockwise direction against the bias of the compression springs 174, storing additional energy in the springs 174. This deflection begins in FIG. 9B and continues in FIG. 9C.

As the drill tube 150A is advanced further into the passage 129, the locking ring 172 remains deflected until the notch 157 in the drill tube 150A aligns with the chamfer 177, as shown in FIG. 9D. When this alignment occurs, the force deflecting the locking ring 172 is temporarily released, allowing the compression springs 174 to expand and return to their relaxed state. This expansion causes locking ring 172 to rotate back toward the locking orientation. The chamfer 177 snaps radially inwardly into the notch 157 under the bias of the compression springs 174. The axial dimension of the locking ring 172 is substantially equal to the axial dimension of the slot 157, such that the axial position of the drill tube 150A is fixed and locked in the guide body 120.

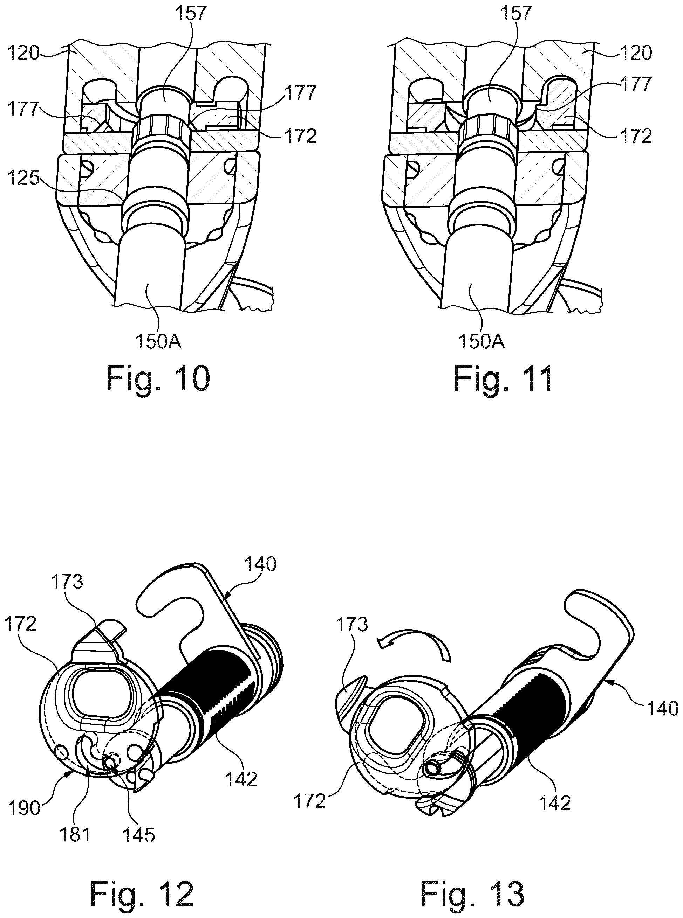

FIG. 10 shows a cross section showing the locking ring 172 in the locking orientation. The portion of the locking ring 172 that engages with the notch 157 is circled. FIG. 11 shows a cross section showing the locking ring 172 in the release orientation. In this condition, no portion of the locking ring 172 extends into the notch 157. As can be appreciated from these two Figures, the drill tube 150A can be removed from the guide body 120 by rotating the switch 173 in the counterclockwise direction against the bias of the compression springs 174. This rotation moves the chamfer 177 out of the notch 157, as shown in FIG. 11. In this state, the drill tube 150A is no longer axially restrained by the locking ring 172 and can be pulled out of the guide body 120.

The drill bits 200A, 200B and 200C each have an outer diameter that corresponds to the inner diameter of the drill tubes 150A, 150B and 150C, respectively. Each of the drill bits 200A, 200B and 200C is long enough to extend from above the drill stop 140 to beyond the distal end of the drill tube 150A when the drill tube 150A is attached to the guide body 120.

After drilling is completed, it is sometimes necessary to remove the drill driver and the universal drill guide 110 from the operating side while leaving the drill tube and drill bit in place. This can be a technical challenge, because the drill stop 140 is engaged with the drill bit as shown in FIG. 1. Therefore, the universal drill guide 110 includes a mechanism that pivots the drill stop 140 counterclockwise and away from the drill bit as the universal drill guide 110 is released from the drill tube. This is accomplished with a camming mechanism 190 that interconnects the locking ring 172 with the shaft 142 of the drill stop 140, as shown in FIGS. 12 and 13.

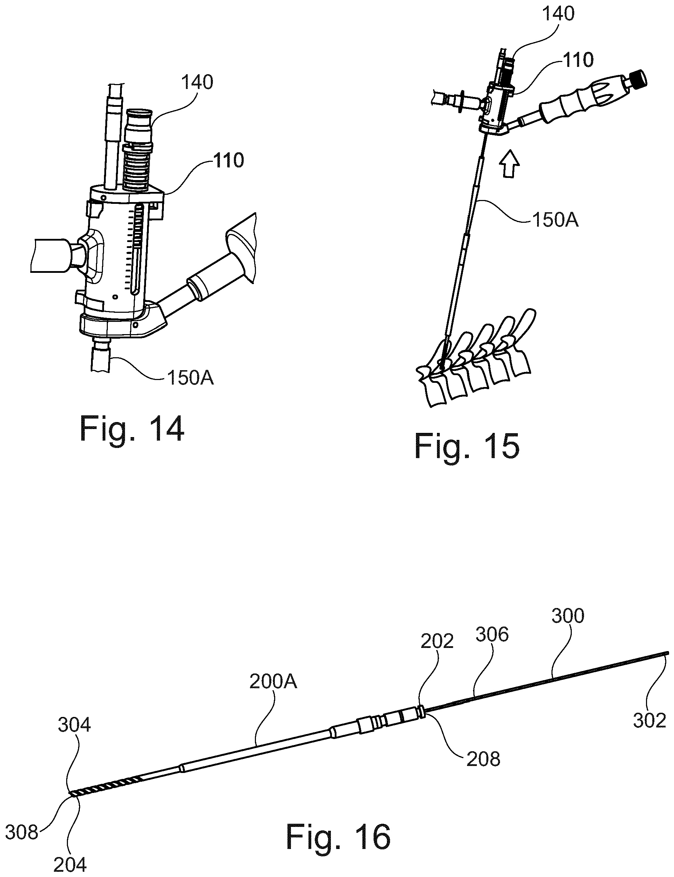

The locking ring 172 includes a cam slot 181 that drives a cam following pin 145 at the bottom of the shaft 142. With this arrangement, rotation of the locking ring 172 clockwise (or left in the Figures) causes the shaft 142 and drill stop 140 to simultaneously rotate counterclockwise (or right in the Figures), moving the drill stop 140 away from the drill bit so that the universal drill guide 110 can be lifted off of the drill tube while the drill tube and drill bit remain in the patient. The process of lifting universal drill guide 110 off of drill tube 150A is illustrated in FIGS. 14 and 15.

It can be seen in FIG. 14 that the switch 173 on the locking ring 172 is rotated clockwise or to the left. This pivots the drill stop 140 counterclockwise or to the right. While disengaging the drill stop from drill bit 200A, the universal drill guide 110 can be lifted off of the drill tube 150A and the drill bit without being inhibited, as shown in FIG. 15.

### Cannulated Drill with K-Wire Retention

The first, second and third drill bits 200A, 200B and 200C are cannulated and have features designed to retain a K-wire, as described previously. The drill bits 200A, 200B and 200C are generally the same, but have a few differences. The outer diameters of the drill bits 200A, 200B and 200C are different, with each outer diameter configured to drill a hole of a different size. The inner diameter of the drill bit 200A is smaller than the inner diameters of the drill bits 200B and 200C, with the inner diameter of the drill bit 200A configured to allow a K-wire having a diameter of 1.0 mm to pass through the drill bit 200A. The inner diameters of the drill bits 200B and 200C are larger, each configured to allow a K-wire having a diameter of 1.5 mm to pass through the drill bits 200B, 200C. The drill bits 200A, 200B and 200C also have different colored indicia on their exterior to assist the user in pairing each drill bit with its corresponding drill tube.

Referring now to FIG. 16, a drill bit assembly with the drill bit 200A will be described in more detail, with the understanding that the same description applies to the drill bits 200B and 200C. The drill bit 200A is shown with a K-wire 300. The K-wire 300 extends through a passage 208 that extends through the drill bit 200A from the proximal end 202 to the distal end 204. The K-wire 300 has a proximal end 302, a distal end 304 and a wire body 306 extending between the proximal and distal ends. The distal end 304 has a sharp pointed end 308 that can be punched into the bone at a selected location. Once the K-wire 300 is punched into the bone, a surgeon can pass a cannulated bone screw, a drill bit, or other instruments over the K-wire and advance it to the selected location to perform an operation.

The K-wire 300 is significantly longer than the drill bit 200A. Therefore, the K-wire 300 can extend through the drill bit 200A with the proximal end 302 of the K-wire projecting proximally and outside of the proximal end 202 of the drill bit 200A. At the same time, the K-wire 300 can extend through the drill bit 200A with the distal end 304 of the K-wire projecting distally and outside of the distal end 204 of the drill bit 200A. The K-wire 300 is releasably securable inside the drill bit 200A as an assembly that allows the K-wire and the drill bit to be drilled into a bone together.

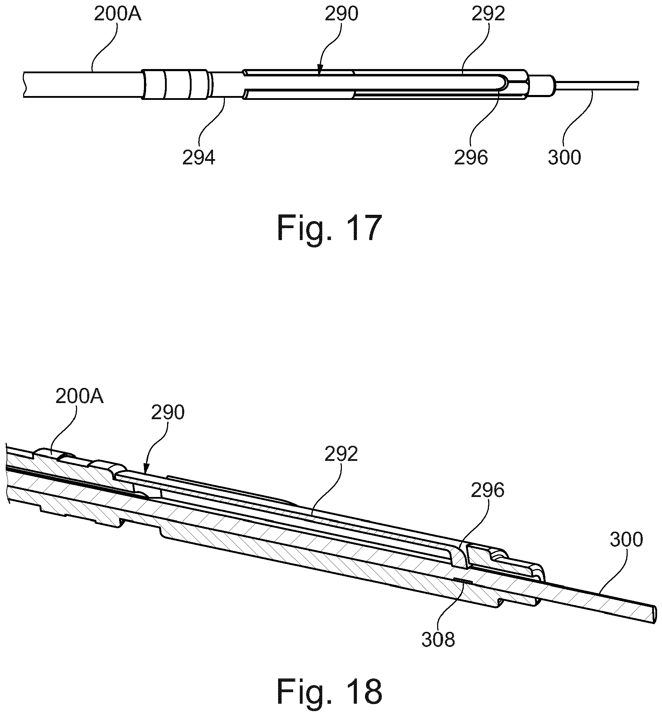

Referring to FIGS. 17 and 18, K-wire 300 is releasably secured to the inside of the drill bit 200A during drilling by a retention mechanism 290 that is built into the drill bit. The retention mechanism 290 is configured to engage with the K-wire 300 to prevent axial advancement of the K-wire relative to the drill bit 200A during drilling. This retention ensures that the K-wire 300 and the drill bit 200A are inserted together and advance the same amount into the bone. The retention mechanism 290 also allows torque applied to the drill bit 200A to be transferred to the K-wire 300. Thus, the K-wire 300 and the drill bit 200A rotate in unison when the retention features are engaged. A torque driver can apply torque to the K-wire 300 and the drill bit 200A in unison to plant the K-wire in the bone and form a pilot hole for a bone screw. Once the pilot hole is drilled, the drill bit 200A can be disengaged from the K-wire 300 and removed from the patient while leaving the K-wire in the bone.

The retention mechanism 290 includes a retention clip 292 configured to releasably engage with a locking groove 308 on an exterior portion of the K-wire 300. The locking grooves and the retention clips according to the present disclosure can have various forms and geometries. For example, the locking groove can extend around a portion of the K-wire, or completely surround the K-wire in a circumferential manner. The retention clip can be clipped onto the exterior of the drill bit, or be formed integrally with the drill bit. The retention clip can also have an inward-extending retention end that can be pressed into the locking groove to limit axial displacement of the K-wire in the drill bit.

The retention clip 292 has a hub portion 294 that attaches the retention clip to the drill bit 200A. The retention clip 292 also has a retention end 296 opposite the hub portion 294. The retention end 296 projects radially inwardly through a sidewall of the drill bit 200 to engage with the locking groove 308 of the K-wire 300.

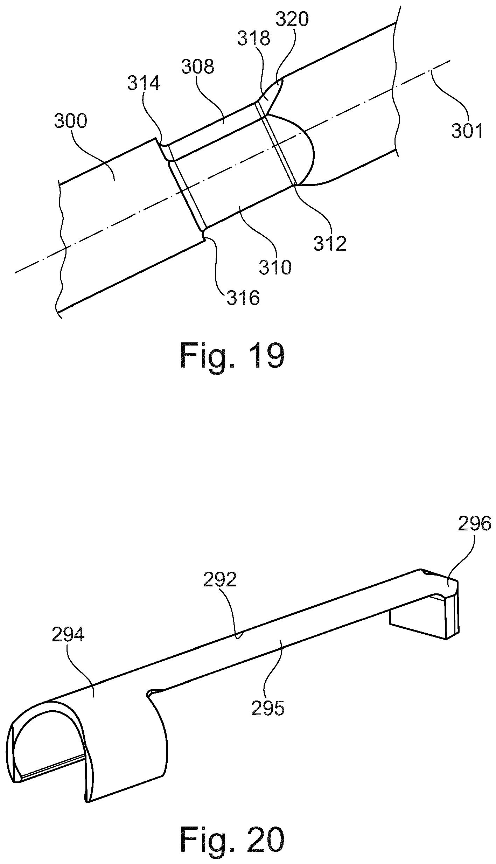

The K-wires according to the present disclosure can have a geometry in the locking groove that allows torque to be transferred from the drill bit/the retention clip to the K-wire. For example, the K-wire can have a flat surface on a portion of its exterior in the groove that engages with a flat edge on the retention end of the retention clip. FIG. 19 shows an example of a K-wire 300 with a locking groove 308 that has a four-sided square-shaped section 310 in the groove. The K-wire 300 can work with a retention clip formed in a drill bit according to any of the previous examples, such as the drill bit 200A.

Locking grooves according to the present disclosure can be bounded by a proximal end wall and a distal end wall. The proximal and distal end walls can have different geometries that control axial displacement of the K-wire relative to the surrounding drill bit. In the example in FIG. 19, the locking groove 308 has a proximal end wall 312 and a distal end wall 314. The distal end wall 314 is substantially perpendicular to a longitudinal axis 301 of the K-wire 300. This forms a stop 316 that can abut on the retention end 296 of the retention clip 292 when the retention clip 292 is engaged in the locking groove 308, thereby preventing relative displacement of the K-wire 300 in the distal direction. In contrast to the distal end wall 314, the proximal end wall 312 consists of inclined surfaces 318 extending at an acute angle relative to the longitudinal axis 301. The inclined surfaces 318 form a ramped section 320 that allows the retention end 296 of the retention clip 292 to gradually deflect outwardly and slide out of the locking groove 308 during withdrawal of the drill bit 200A from the K-wire 300 after drilling is completed.

The retention clip 292 is operable in a locked mode and a released mode. In the locked mode, the retention end 296 is pressed and held inwardly in the locking groove, as shown in FIG. 18. This locks the axial position of the K-wire 300 relative to the drill bit 200 during drilling. After drilling is complete, the inward force on the retention end 296 can be removed, leaving the retention clip 292 in the released mode. In the released mode, it is possible to move the drill bit 200 in a proximal direction relative to the K-wire 300 until the drill bit is completely removed from the K-wire. This allows the drill bit 200A to be withdrawn from a patient while leaving the K-wire 300 in the patient for further use.

Referring to FIG. 20, the retention clip 292 has a partially-cylindrical hub portion 294 configured to clip onto the rounded exterior of a drill bit, similar to a pocket clip on a pen. The retention clip 292 also has a flexible arm 295 between hub portion 294 and a retention end 296. The flexible arm 295 allows the retention end 296 to flex radially outwardly under stored energy as the drill bit is withdrawn from the K-wire. The retention end 296 remains deflected in an outward position as the drill bit is removed from the K-wire. Once the drill bit is removed from the K-wire, the retention end 296 snaps back in the radially inward direction to its relaxed state shown in FIG. 20.

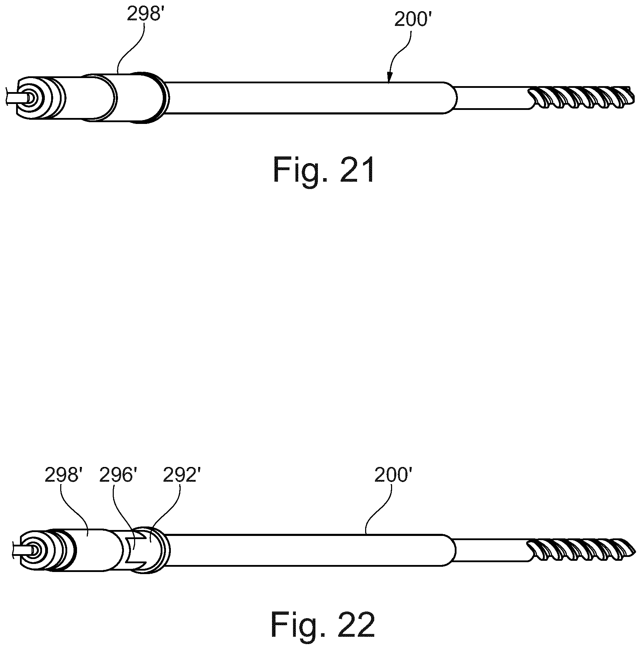

Force can be applied to the retention ends of the retention clips in a radially inward direction to press and hold the retention clips in the engaged mode with the locking groove. The inward force can be applied in a number of ways. FIGS. 21 and 22 show another example of a drill bit assembly with a drill bit 200' and a movable sleeve 298'. The movable Sleeve 298' is slidable over the exterior of the drill bit 200' between a first position and a second position. In the first position (FIG. 21), the sleeve 298' covers the retention end 296' and applies an inward force to it to hold the retention clip 292' in the engaged mode. In the second position (FIG. 22), the sleeve 298' is moved off of the retention end 296', allowing the retention end 296' to flex outwardly to permit disengagement of the retention clip 298' from the locking groove on a K-wire 300.

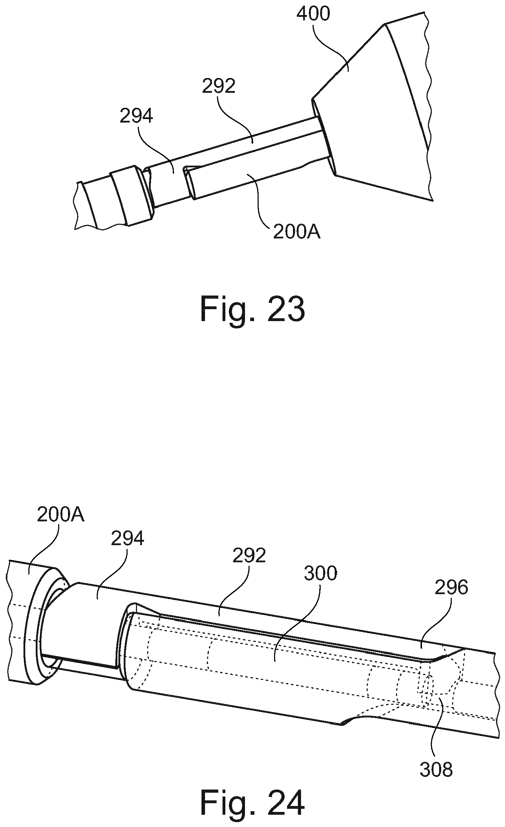

In other examples, a separate instrument can be used to lock the retention clip into engagement with the K-wire. FIGS. 23 and 24 show an example in which a retention clip 292 of a drill bit 200A is maintained in the engaged mode by a drill driver 400. The drill driver 400 is clamped over the retention end 296 of the retention clip 292 and applies external force to maintain the retention clip 292 in the engaged mode in a four-sided locking groove 308 of a K-wire 300.

### Drill Removal Tool

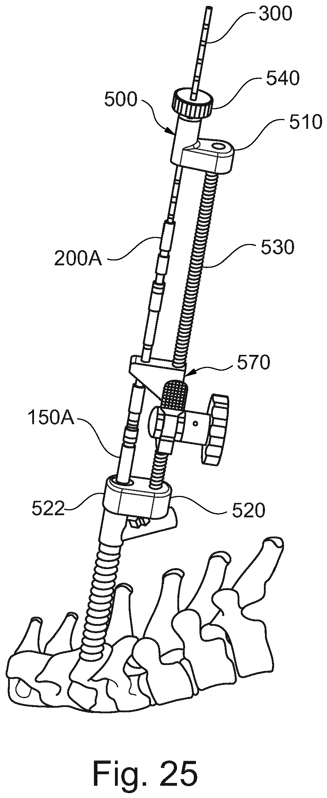

Referring to FIGS. 25-32, a drill removal tool 500 is shown according to one example. The drill removal tool 500 is designed to remove a cannulated drill bit from the bone after a hole is drilled, while leaving the K-wire in place in the bone. Once the drill bit is removed from the K-wire, a bone screw can be advanced over the K-wire and driven into the screw hole. The drill removal tool 500 can be used with any combination of drill bit, drill tube and K-wire. For purposes of this description, the drill removal tool 500 will be described in use with the same drill bit 200A, drill tube 150A and K-wire 300 described previously.

The drill removal tool 500 includes a first support end 510, a second support end 520 and a toothed rack 530 extending between the first support 510 end and second support end 520. The first support end 510 includes a wire clamp 540 operable to clamp onto the K-wire 300. The second support end 520 include a C-shaped base 522 configured to fit snugly around the drill tube 150A. A drill bit remover 570 is axially displaceable on the toothed rack 530 between the first support end 510 and the second support end 520.

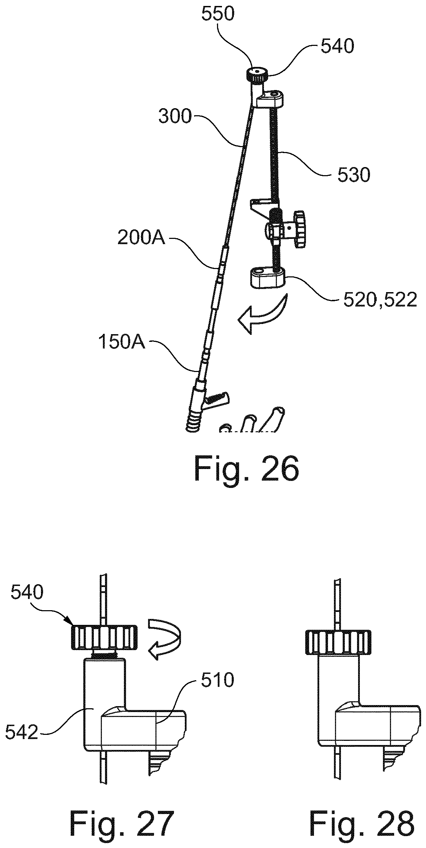

Referring to FIGS. 26-29, the wire clamp 540 is configured to be passed over the exposed end of the K-wire 300 in an unlocked state, and subsequently clamp the K-wire 300 in a locked state. The wire clamp 540 includes a hollow housing 542 which extends from the first support end 510. The hollow housing 542 defines a clamp passage 544 having a proximal end 546, a distal end 548 and a converging section 549 between the proximal end 546 and the distal end 548. The proximal end 546 has an internal thread 547. A knob 550 includes a dial 552 and a shaft 554 with an external thread 557 that mates with the internal thread 547 in the clamp passage 544. The knob 550 defines a through bore 558 that axially receives a clamping pin 560. The clamping pin 560 defines a through passage 562 and a wedge shaped collet 564 at its distal end 566. The collet 564 has an outer diameter that gradually decreases toward the distal end 566, forming a cone-shaped projection that conforms to the shape of the converging section 549. The through passage 562 has an inner diameter adapted to receive the K-wire 300.

The knob 550 is rotatable about a knob axis 551 between the unlocked state and the locked state. The knob axis 551 aligns coaxially with the through bore 558 and the through passage 562. FIG. 27 shows the knob 550 in the unlocked state, and FIG. 28 shows the knob after it is rotated to the locked state. The internal thread 547 and the external thread 557 promote axial displacement of the knob 550 when the knob is rotated. The clamping pin 560 is axially fixed in the knob 550, with the distal end 556 of the knob 550 abutting on the collet 564. In this arrangement, the knob 550 and the clamping pin 560 move axially and in unison in the clamp housing 544 when the knob is rotated. The internal and the external threads 547, 557 are oriented so that rotation of the dial 552 in a clockwise direction CW causes the knob 550 and the collet 564 to move distally into the converging section 549. The tapered shape of the converging section 549 exerts inward force on the collet 564 as the clamping pin 560 moves distally, compressing the collet 564 so that it locks the K-wire 300 in a locked state.

The drill removal tool 500 is attachable over the K-wire 300, the drill bit 200A and the drill tube in two steps. In a first step, the wire clamp 540 is passed over the K-wire. To pass the wire clamp 540 over the K-wire 300, the knob 550 is rotated counterclockwise to the unlocked state so that the collet 564 is not compressed in the converging section 549 of the clamp passage 544. Once the proximal end of the K-wire 300 passes through the wire clamp 540, the second support end 520 and the C-shaped base 522 are pivoted toward the drill tube 150A in the direction shown by the curved arrow in FIG. 26. The C-shaped base 522 is pivoted until the opening 523 in the C-shaped base receives the drill tube 150A in a snug fit.

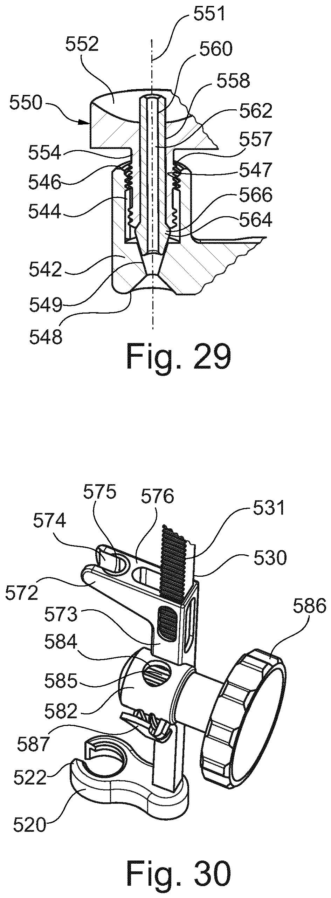

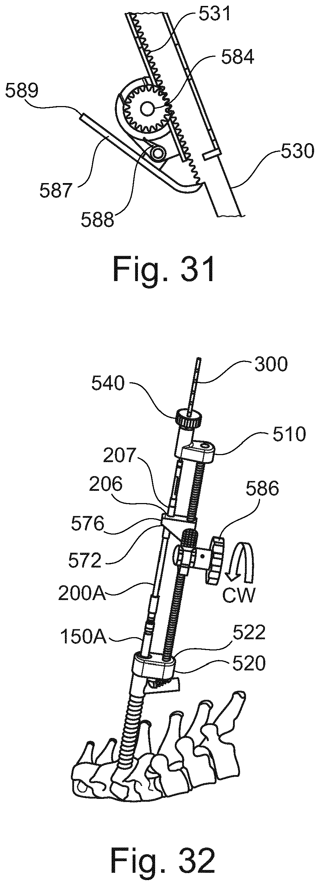

Referring to FIG. 30, the drill bit remover 570 includes a fork-shaped anvil 572 that defines a through slot 574. The through slot 574 aligns with the clamp passage 544 and the opening 523, forming a straight passage through all three parts of the drill removal tool 500. The anvil 572 has a flat fork-lift surface 576 above through slot 574. The through slot 574 and the fork-lift surface 576 are configured to engage with the drill bit 200A beneath the stop surface 206, as mentioned earlier. The through slot 574 has a rounded end 575 with a diameter that is slightly larger than the reduced diameter section 205 on the drill bit 200A, but smaller than the enlarged diameter section 207. Therefore, the anvil 572 is configured to receive the reduced diameter section 205 of the drill bit 200A into the through slot 574, with the fork-lift surface 576 positioned beneath or distally with respect to the stop surface 206. In this position, the fork-lift surface 576 abuts on the enlarged diameter section 207 at the stop surface 206, as shown in FIG. 32.

The drill bit remover 570 includes a sleeve 573 connected to the anvil 572. The sleeve 573 surrounds the rack 530 and interconnects the anvil 572 with the C-shaped base 522. A pinion housing 582 extends from one side of the sleeve 573 and contains a pinion 584. The pinion 584 has a plurality of gear teeth 585 that mesh or engage with the teeth 531 on the rack 530 through an opening between the pinion housing 582 and the rack 530. A wheel handle 586 is attached to the pinion 584 and extends outside of the pinion housing 582. The wheel handle 586 and the pinion 584 are rotatable in unison relative to the pinion housing 582. In this arrangement, the wheel handle 586 can be rotated to move the drill bit remover 570 up or down along the rack 530.

Referring to FIGS. 30 and 31, a spring loaded pawl 587 releasably engages with the teeth 531 on the rack 530. The pawl 587 is biased into engagement with the teeth 531 by a spring 588. Engagement of the pawl 587 with the teeth 531 occurs automatically after the drill bit remover 570 is displaced along the rack 530, and serves to lock the position of the drill bit remover 570 relative to the rack 530 to maintain the position of the anvil 572. The pawl 587 is pivotable out of engagement with the teeth 531 against the bias of the spring 588 to release the pawl 587 and allow the drill bit remover 570 to be moved on the rack 530. The pawl 587 can be pivoted out of engagement with the teeth 531 by depressing a tab 589 that extends from the pawl 587. If desired, the strength of the spring 588 can be designed to hold the pawl 587 against the rack 530 but allow the pawl 587 to ride along the rack 530 when the wheel handle 586 is turned, in the manner of a ratchet. Alternatively, the strength of the spring 588 can be selected so that the pawl 587 will not disengage from the teeth 531 when the wheel handle 586 is turned, and only disengage from the teeth 531 when the user depresses the tab 589.

The pinion 584 is positioned on the left side of the rack 530 when facing the wheel handle 586. In this arrangement, rotation of the wheel handle 586 in the clockwise direction CW raises the anvil 572 upwardly, or toward the first support end 510. This causes the fork-lift surface 576 to bear upwardly or proximally against the stop surface 206 on the drill bit 200A, displacing the drill bit 200A in the proximal direction. Therefore, to remove the drill bit 200A from the patient, without removing the K-wire 300, the user first locks the wire clamp 540 to axially fix the K-wire 300. Then, the user rotates the wheel handle 586 clockwise to move the drill bit 200A in the proximal direction relative to the K-wire 300. This has the effect of withdrawing the drill bit 200A from the patient while not displacing the K-wire 300 and keeping the K-wire in place. The wheel handle 586 is rotated until the distal end 204 of the drill bit 200A is removed from the patient. Once the drill bit 200A is no longer in the patient, the wire clamp 540 is unlocked, and the C-shaped base 522 is detached from the drill tube 150A. This allows the drill removal tool 500 to once again move along the K-wire 300. The drill bit removal tool 500 is then lifted off of the K-wire 300, with the fork-lift surface 576 supporting the drill bit 200A such that the drill bit 200A is also removed from the K-wire 300. After the drill bit removal tool 500 is removed from the K-wire 300, the drill tube 150A can be removed from the patient, as well any tissue dilators. In the present example, the drill tube 150A extends through multiple telescopic dilators, the outermost dilator D being visible in FIG. 32.

In an alternate MIS technique, the drill bit 200A can be drilled into the bone without the K-wire 300. In such instances, the drill removal tool 500 can be used to insert the K-wire 300 into the bone through the drill bit 200A. To begin this technique, a universal drill guide such as the embodiment previously described is attached to a navigation unit and calibrated. The drill stop height is set and the appropriate drill tube is attached to the universal drill guide. An obturator is then inserted into the universal drill guide and drill tube and locked in place. The universal drill guide and obturator are then probed to the desired drilling position in the patient. The obturator is then removed from the universal drill guide and drill tube, and replaced with a cortical punch. The cortical punch is used to create a start hole in the cortical layer, and then removed. The appropriate drill bit is then attached to a driver, inserted into the universal drill guide, and advanced through the drill tube to drill a hole into the bone. Once the drill bit is drilled into bone, the universal drill guide is detached and removed from the drill tube, leaving the drill bit and the drill tube in place.

A tissue dilator D is attached to a dilator handle and advanced over the drill tube until it contacts the tissue surrounding the drill tube. The dilator is then pushed and rotated to dilate tissue. A tissue protector P is then advanced over the dilator. A K-wire can be then be inserted into the bone through the drill bit.

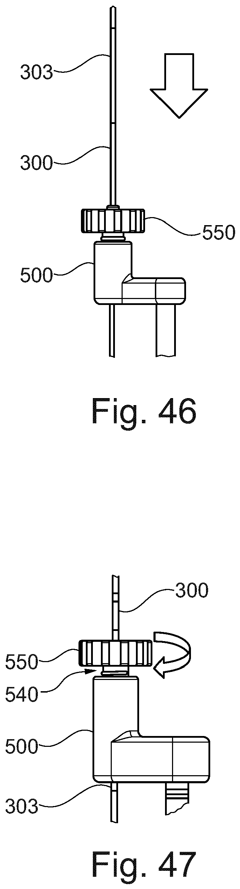

Referring to FIGS. 46-52, one process for inserting a K-wire 300 through the drill bit 200A is shown. In a first step, the K-wire 300 is loaded into drill removal tool 500. The knob 550 is rotated to an unlocked position, and the K-wire 300 is inserted through the knob in the direction shown by the arrow in FIG. 46. The K-wire 300 is advanced through the knob 550 until a long marking 303 on the K-wire is fully covered by the drill removal tool 500. Once the K-wire 300 is advanced through the drill removal tool 500 to the appropriate position, the knob 550 is rotated to a locked position as shown by the arrow in FIG. 47 to lock the K-wire in the wire clamp 540.

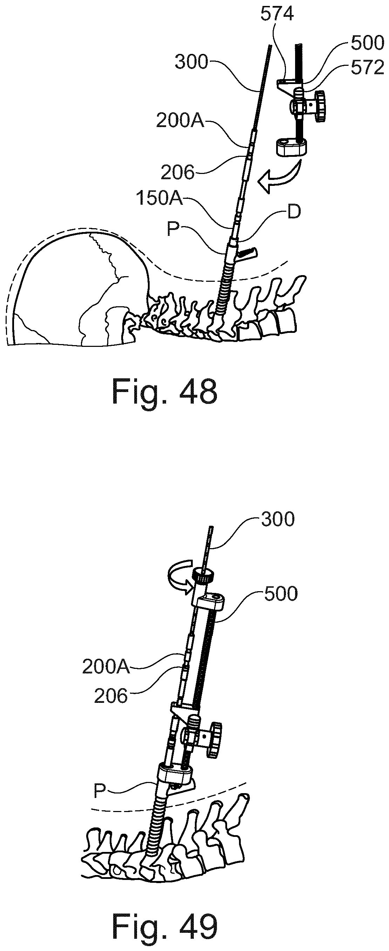

The drill removal tool 500 and the K-wire 300 are positioned over the proximal end 202 of the drill bit 200A. The K-wire 300 is then guided down into the drill bit 200A. The bottom portion of the drill removal tool 500 is pivoted toward the drill bit 200A and dilator D as shown in FIG. 48 until the drill bit is received in the through slot 574 of the anvil 572. The drill removal tool 500 is pivoted while making sure that the anvil 572 is positioned beneath the stop surface 206. Once the drill removal tool 500 is mounted to the drill bit 200A and dilator D, the knob 550 is rotated to the unlocked position in the direction shown in FIG. 49.

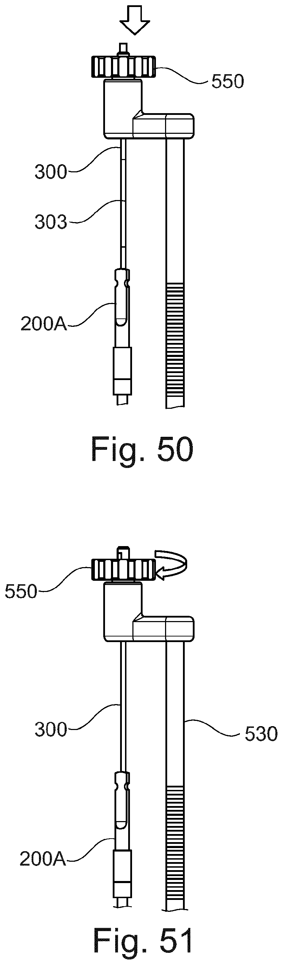

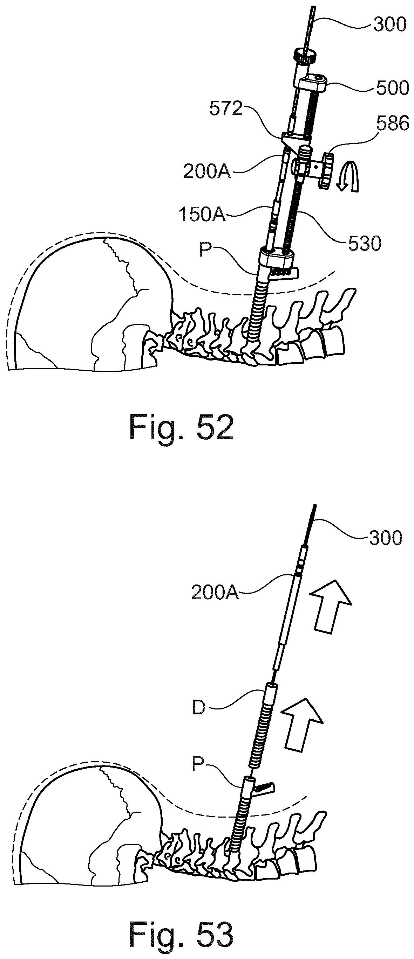

The K-wire 300 is advanced downwardly into the drill bit 200A as shown in FIG. 50. Once the long marking 303 is completely covered by the drill bit 200A, the knob 550 is rotated to the locked position as shown in FIG. 51. The wheel handle 586 is then rotated, as shown in FIG. 52, to move the anvil 572 upwardly relative to the toothed rack 530 and pull the drill bit 200A out of the bone. Once the drill bit 200A is pulled out of the bone, the drill removal tool 500 and the drill bit can be lifted and removed. The drill tube 150A and dilator D can then be removed from protector P, as shown in FIG. 53.

### Bone Screw Driver with K-wire Retention

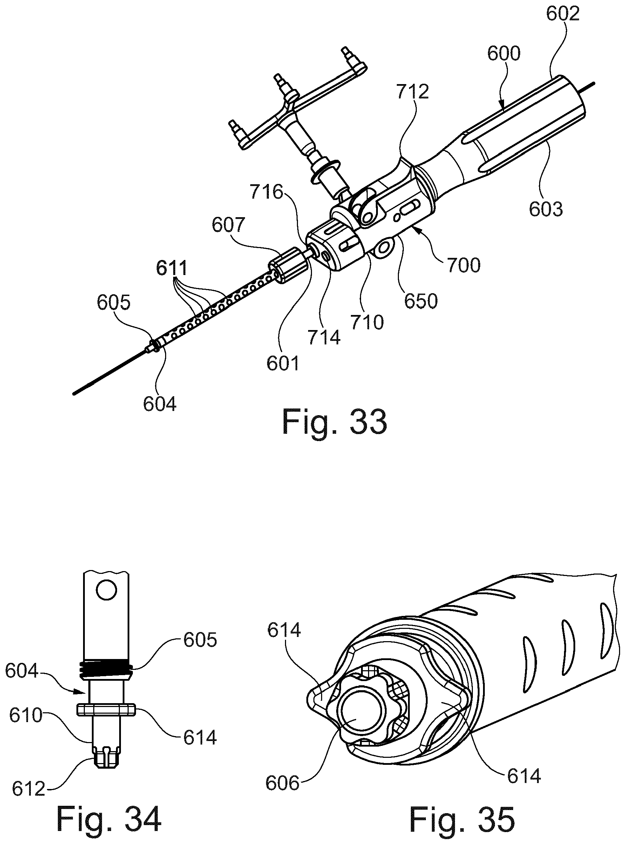

Referring to FIGS. 33-41, a bone screw driver assembly with a bone screw driver 600 is shown according to one example. The bone screw driver 600 is designed to advance a bone screw over a K-wire and drive the bone screw into the bone while preventing forward (i.e. distal) advancement of the K-wire. To accomplish this, the bone screw driver assembly features a K-wire retention module 700. For purposes of this description, the bone screw driver 600 and the K-wire retention module 700 will be described in use with the same K-wire 300 described previously.

The bone screw driver 600 has a shaft 601 that defines a proximal end 602, a distal end 604 opposite the proximal end 602, and a longitudinal through passage 606 between the proximal 602 and distal ends 604. The passage 606 is configured such that the bone screw driver 600 can be passed over the proximal end of the K-wire 300 and advanced toward the distal end of the K-wire 300. The proximal end 602 has an attachment mechanism (not visible) over which a handle 603 is attached. The attachment mechanism can be any suitable structure for receiving a handle, including but not limited to a hex shaped shaft. The handle 603 is configured to be gripped by a user and rotated to operate the bone screw driver 600, much like a conventional screw driver. The distal end 604 has an external thread 605 configured to mate with an internal thread in a rod receiving component of a pedicle screw assembly. A knob 607 is provided on the shaft 601 to facilitate rotation of the shaft 601 to thread the external thread 605 into a rod receiving component.

Referring to FIGS. 34 and 35, the distal end 604 has a driver tip 610. The driver tip 610 has a hexalobular extension 612 that fits into a similarly shaped recess in the head of the bone screw. The driver tip 610 also has a pair of tangs 614 located proximally relative to extension 612. The tangs 614 are configured to slide into diametrically opposed slots in a rod receiving component when the external thread 605 is threaded into the internal thread of the rod receiver component. In this arrangement, the tangs 614 occupy the location where a fixation rod will be located.

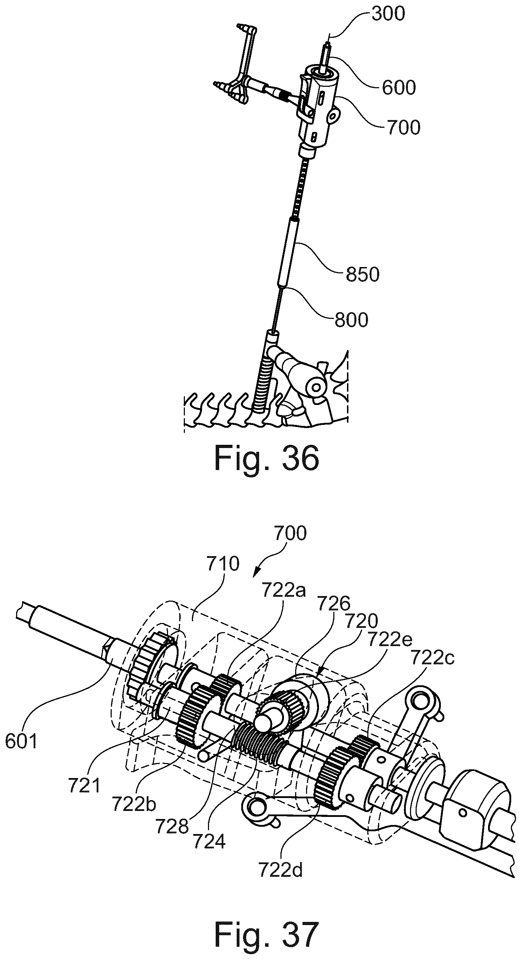

The K-wire retention module 700 includes a housing 710 having a proximal end 712, a distal end 714, and a passage 716 extending between the proximal end 712 and the distal end 714. The passage 716 aligns with the passage 606 of the bone screw driver 600. In this arrangement, the bone screw driver 600 and the K-wire retention module 700 can be advanced over the K-wire 300 as a unit. FIG. 36 shows the bone screw driver 600 (without the handle 603 attached) in the process of being advanced over an implanted K-wire 300. The driver tip 610 is secured to a cannulated polyaxial screw assembly 800 and a tab protector sleeve 850 that also pass over the K-wire 300.

Referring to FIG. 37, the K-wire retention module 700 has a roller assembly 720 contained within the housing 710. The roller assembly 720 is operable in a disengaged mode and an engaged (or drive) mode. In the disengaged mode, the roller assembly 720 allows the K-wire retention module 700 and the bone screw driver 600 to advance freely along the length of the K-wire 300. In the engaged mode, the roller assembly 720 engages with K-wire 300 and feeds the K-wire 300 through the housing 710 in a proximal direction as the bone screw driver 600 advances the polyaxial screw assembly 800 in a distal direction.

The roller assembly 720 includes a first spur gear 722a attached to the shaft 601 of the bone screw driver 600, and a second spur gear 722b mated with the first spur gear 722a. The roller assembly 720 also includes a third spur gear 722c on the shaft 601 that is mated with a fourth spur gear 722d. The second spur gear 722b and the fourth spur gear 722d are attached to a secondary shaft 721 that extends parallel to the shaft 601. The secondary shaft 721 has a worm gear 724 mated with a fifth spur gear 722e. The fifth spur gear 722e is attached to a first roller 726, which is fixed to the fifth spur gear 722e so that the fifth spur gear 722e and the first roller 726 rotate in unison. A second roller 728 is positioned adjacent to the first roller 726 at a position to engage with the K-wire 300 on a side opposite the first roller 726.

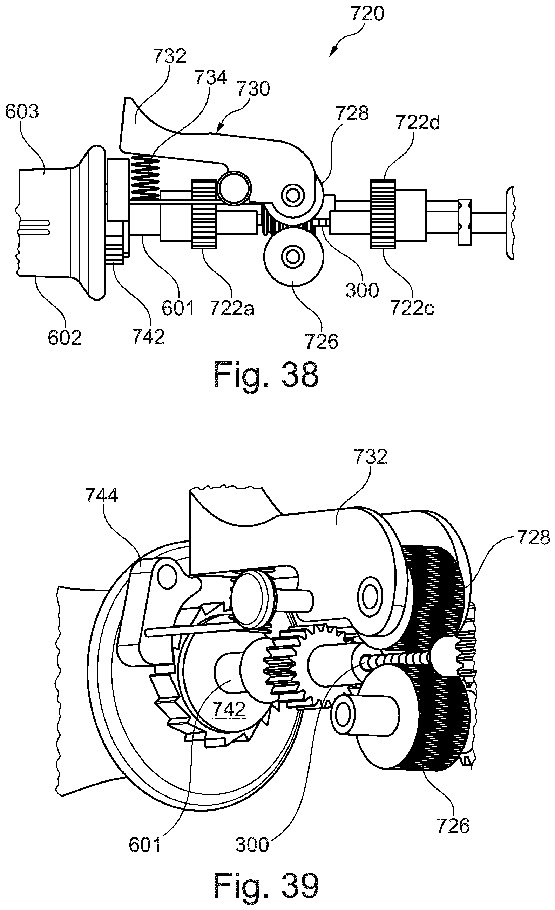

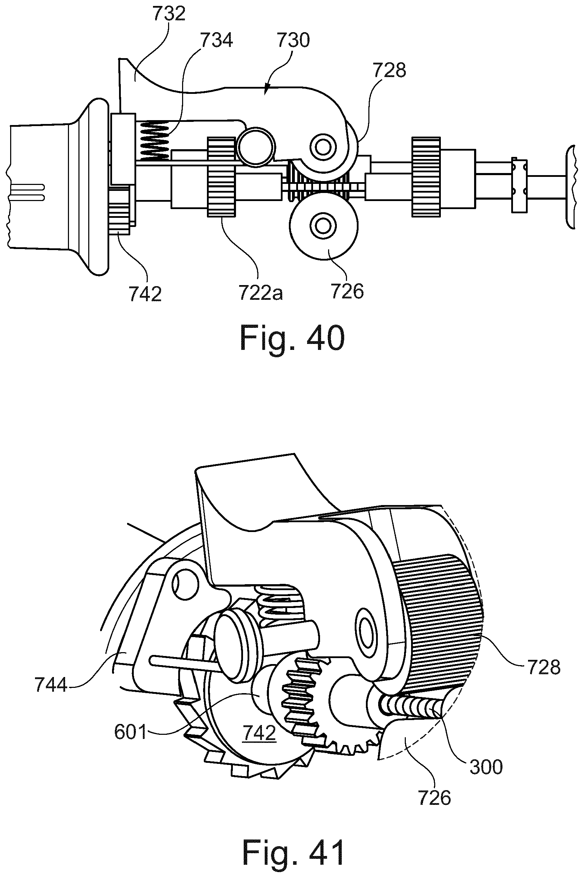

Referring to FIGS. 38-41, engagement and disengagement of the roller assembly 720 is controlled by a lever assembly 730. The lever assembly 730 includes a spring-loaded lever 732 that is biased toward the engaged mode by a compression spring 734. FIG. 38 shows components of the roller assembly 720 about to be advanced over the K-wire 300. The lever 732 is positioned in the engaged mode by the spring 734, which is fully expanded. In this mode, the first and the second rollers 726, 728 are positioned close together, with little or no clearance between them. To advance the roller assembly 720 over the K-wire 300, the lever 732 is pressed inwardly toward the K-wire, as shown in FIG. 40. This moves the first and the second rollers 726, 728 apart, allowing the roller assembly 720 to advance over the K-wire 300. Once the K-wire 300 is received between the first and the second rollers 726, 728, the lever 732 can be released to allow the roller assembly 720 to return to the engaged mode under the bias of the spring 734, which positions the rollers in direct engagement with the K-wire.

The polyaxial screw assembly 800 is driven into the bone over the K-wire 300 by applying clockwise torque to the proximal end 602 of the bone screw driver 600. When clockwise torque is applied to the proximal end 602 of the screw driver 600 with the roller assembly 720 in the engaged mode, the roller assembly 720 will feed the K-wire 300 through the housing 710 in the proximal direction. Clockwise rotation of the shaft 601 rotates the first spur gear 722a and the third spur gear 722c in a clockwise direction, which in turn impart torque to the secondary shaft 721 through the second spur gear 722c and the fourth spur gear 722d. The secondary shaft 721 and the worm gear 724 rotate in a counterclockwise direction, which imparts torque to the fifth spur gear 722e. The fifth spur gear 722e drives the first roller 726 in a first direction. The second roller 728 is biased into engagement with the K-wire 300 and rotates in a second direction opposite first direction. The outer surfaces of the first and the second rollers 726, 728 grip the surface of the K-wire 300 to draw the K-wire in the proximal direction relative to the housing 710, so that the K-wire 300 is fed proximally as the polyaxial screw assembly 800 is driven distally into the bone.

Distal feeding of the K-wire 300 through the housing 710 is prevented by a ratchet wheel 742 and a pawl 744. The pawl 744 engages with the ratchet wheel 742, as shown in FIG. 39, to prevent the shaft 601 from being rotated counterclockwise relative to the housing 710, which would rotate the first and the second rollers 726, 728 in a reverse direction that feeds the K-wire distally. The pawl 744 can be pivoted out of engagement with the ratchet wheel 742 by pressing the lever 732 inwardly against the spring 724, as shown in FIG. 41. This switches the roller assembly 720 to the disengaged mode and releases the K-wire 300, allowing the bone screw driver 600 and the K-wire retention module 700 to be removed from the K-wire 300 without risk of distally advancing the K-wire 300.

K-wire retention modules according to the present disclosure can be modular units that are detachably connectable to different types of instruments, including but not limited to instruments for tapping and driving. In the present example, the K-wire retention module 700 is detachably connected to the bone screw driver 600 with a quick fit connection 650 represented in FIG. 33. The shaft 601 of the bone screw driver 600 snaps into the housing 710 using the quick fit connection 650, which can be a hex drive, ¼ inch drive or AO drive that controls rotation.

Bone screw drivers according to the present disclosure can include various types of indicia for aiding the insertion of a bone screw. For example, the shaft 601 can have spaced lines that provide depth markings, similar to those on the universal drill guide 110. Depth markings can provide the user with a visual indication of the depth to which the tip of the bone screw is advanced. Bone screw drivers can also include various features to aid in sterilization. For example, the shaft 601 has a series of apertures 611 that allows steam to access the inside of the shaft 601 during autoclaving and cleaning.

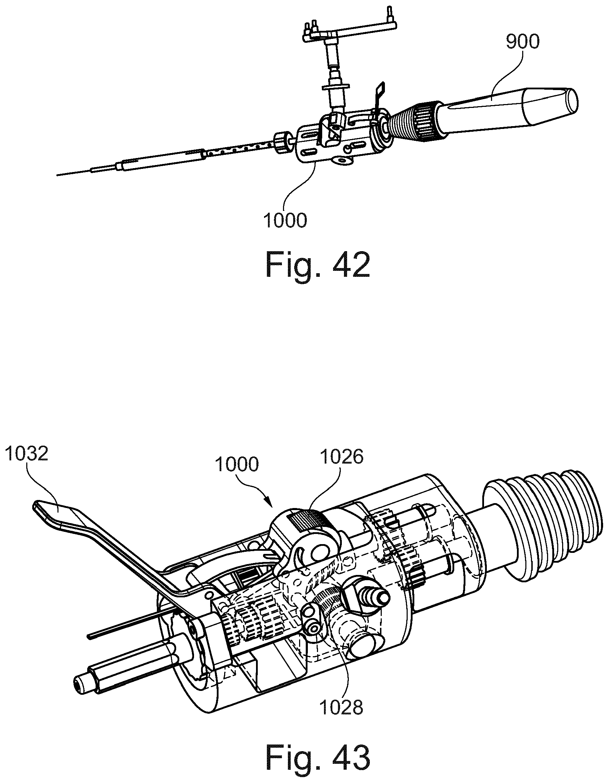

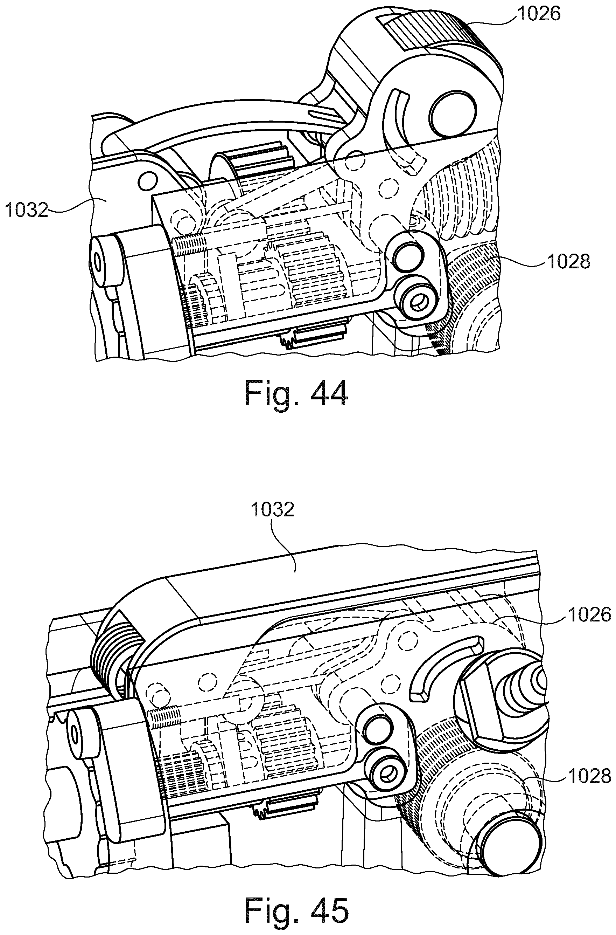

Figures 42-45 show an alternate bone screw driver 900 and K-wire retention module 1000 according to another embodiment. The K-wire retention module 1000 is similar to the K-wire retention module 700, but features a pinch lever 1032 and pinch rollers 1026, 1028. The pinch lever 1032 is normally in an open position to separate the pinch rollers 1026, 1028, as shown in FIGS. 43 and 44. The pinch lever 1032 can be moved to a closed position, as shown in FIG. 45, to engage with the rollers against the K-wire 300. The pinch lever 1032 can only stay closed (i.e. the drive can only remain engaged) when the K-wire 300 is positioned between the pinch rollers 1026, 1028.

The instruments described herein can be manufactured using various materials, including but not limited to various alloys of stainless steel. Alloy grade can be selected based on desired strength, hardness, corrosion resistance, galling properties and other performance criteria.

Accordingly, it is intended that the appended claims cover all such variations as fall within the scope of the present disclosure.

## Claims

1. A universal drill guide system (100) comprising:
a universal drill guide (110);
a drill tube (150) insertable at least partly into the universal drill guide (110); and
a drill bit (200) insertable into the drill tube (150),
wherein the universal drill guide (110) comprises
a drill stop (140) configured to abut on a stop surface (206) of the drill bit (200) to limit a drilling depth of the drill bit (200), and
a locking ring (172) rotatable between a locking orientation, in which the drill tube (150) inserted into the universal drill guide (110) is locked by the locking ring (172), and a release orientation, in which the drill tube (150) is releasable from the locking ring (172), **characterized by**
a camming mechanism (190) configured to pivot the drill stop (140) away from the stop surface (206) of the drill bit (200) as the locking ring (172) is rotated into the release orientation.

2. The universal drill guide system (100) according to claim 1, wherein the camming mechanism (190) includes a cam slot (181) formed on the locking ring (172) and a cam following pin (145) formed on a distal end of a shaft (142) of the drill stop (140).

3. The universal drill guide system (100) according to claim 2, wherein the cam slot (181) and the cam following pin (145) are configured such that a rotation of the locking ring (172) clockwise causes the drill stop (140) to simultaneously rotate counterclockwise, pivoting the drill stop (140) away from the stop surface (206) of the drill bit (200).

4. The universal drill guide system (100) according to any one of claim 1 to 3, wherein a compression spring (174) exerts a biasing force on the locking ring (172) to bias the locking ring (172) in the locking orientation.

5. The universal drill guide system (100) according to clam 4, wherein a switch (173) is manually operable to rotate the locking ring (172) from the locking orientation to the release orientation against the biasing force of the compression spring (174).

6. The universal drill guide system (100) according to any one of claim 1 to 5, wherein the drill stop (140) comprises a stop plate (144) extending from a/ the shaft (142) of the drill stop (140) transversely, the stop plate (144) comprising a slot (146) having a slot dimension configured to permit a distal section of the drill bit (200) to pass through the stop plate (144), until the stop surface (206) of the drill bit (200) abuts on the stop plate (144).

7. The universal drill guide system (100) according to any one of claim 1 to 6, wherein the universal drill guide (110) comprises a guide body (120), and the shaft (142) of the drill stop (140) is configured to be raised or lowered with respect to the guide body (120).

8. The universal drill guide system (100) according to any one of claim 1 to 7, wherein the universal drill guide (110) comprises a lock (126) configured to engage with a/ the shaft (142) of the drill stop (140) to lock the shaft (142) with respect to the guide body (120) by exerting a biasing force.

9. The universal drill guide system (100) according to claim 8, wherein the shaft (142) of the drill stop (140) includes circumferential grooves (143) to engage with the lock (126).

10. The universal drill guide system (100) according to claim 8 or 9, wherein the lock (125) includes a release button (127) configured to be pressed inwardly to disengage the lock (126) from the shaft (142) of the drill stop (140).

11. The universal drill guide system (100) according to any one of claim 1 to 10, wherein the universal drill guide (110) includes a first set of indicia (128) and a second set of indicia (148) for indicating a selected depth.

12. The universal drill guide system (110) according to claim 11, wherein the first set of indicia (128) includes a vertical series of markings (128a) on the guide body (120) and the second set of indicia (148) includes a vertical series of markings (148a) on the shaft (142) of the drill stop (140).

## Patentansprüche

1. Universalbohrerführungssystem (100), mit
einer Universalbohrerführung (110);
einem Bohrerrohr (150), das zumindest teilweise in die Universalbohrerführung (110) einsetzbar ist; und
einer Bohrkrone (200), die in das Bohrerrohr (150) einsetzbar ist,
wobei die Universalbohrerführung (110)
einen Bohreranschlag (140), der so konfiguriert ist, dass er an einer Anschlagfläche (206) der Bohrkrone (200) anliegt, um eine Bohrtiefe der Bohrkrone (200) zu begrenzen, und
einen Verriegelungsring (172) aufweist, der zwischen einer Verriegelungsausrichtung, in der das in die Universalbohrerführung (110) eingesetzte Bohrerrohr (150) durch den Verriegelungsring (172) verriegelt ist, und einer Freigabeausrichtung, in der das Bohrerrohr (150) von dem Verriegelungsring (172) lösbar ist, drehbar ist, **gekennzeichnet durch**
einen Nockenmechanismus (190), der so konfiguriert ist, dass er den Bohreranschlag (140) von der Anschlagfläche (206) der Bohrkrone (200) wegschwenkt, wenn der Verriegelungsring (172) in die Freigabeorientierung gedreht wird.

2. Universalbohrerführungssystem (100) nach Anspruch 1, wobei der Nockenmechanismus (190) einen Nockenschlitz (181), der an dem Verriegelungsring (172) ausgebildet ist, und einen Nockenfolgestift (145), der an einem distalen Ende eines Schaftes (142) des Bohreranschlags (140) ausgebildet ist, enthält.

3. Universalbohrerführungssystem (100) nach Anspruch 2, wobei der Nockenschlitz (181) und der Nockenfolgestift (145) derart konfiguriert sind, dass eine Drehung des Verriegelungsrings (172) im Uhrzeigersinn eine gleichzeitige Drehung des Bohreranschlags (140) gegen den Uhrzeigersinn bewirkt, wodurch der Bohreranschlag (140) von der Anschlagfläche (206) der Bohrkrone (200) weggeschwenkt wird.

4. Universalbohrerführungssystem (100) nach einem der Ansprüche 1 bis 3, wobei eine Druckfeder (174) eine Vorspannkraft auf den Verriegelungsring (172) ausübt, um den Verriegelungsring (172) in die Verriegelungsausrichtung vorzuspannen.

5. Universalbohrerführungssystem (100) nach Anspruch 4, wobei ein Schalter (173) manuell betätigbar ist, um den Verriegelungsring (172) gegen die Vorspannkraft der Druckfeder (174) aus der Verriegelungsausrichtung in die Freigabeausrichtung zu drehen.

6. Universalbohrerführungssystem (100) nach einem der Ansprüche 1 bis 5, wobei der Bohreranschlag (140) eine Anschlagplatte (144) aufweist, die sich von einem/dem Schaft (142) des Bohreranschlags (140) quer erstreckt, wobei die Anschlagplatte (144) einen Schlitz (146) mit einer Schlitzabmessung hat, die so konfiguriert ist, dass ein distaler Abschnitt der Bohrkrone (200) durch die Anschlagplatte (144) hindurchgehen kann, bis die Anschlagfläche (206) der Bohrkrone (200) an der Anschlagplatte (144) anliegt.

7. Universalbohrerführungssystem (100) nach einem der Ansprüche 1 bis 6, wobei die Universalbohrerführung (110) einen Führungskörper (120) aufweist und der Schaft (142) des Bohreranschlags (140) so konfiguriert ist, dass er in Bezug auf den Führungskörper (120) angehoben oder abgesenkt werden kann.

8. Universalbohrerführungssystem (100) nach einem der Ansprüche 1 bis 7, wobei die Universalbohrerführung (110) eine Verriegelung (126) aufweist, die so konfiguriert ist, dass sie mit einem/dem Schaft (142) des Bohreranschlags (140) in Eingriff kommt, um den Schaft (142) in Bezug auf den Führungskörper (120) zu verriegeln, indem eine Vorspannkraft ausgeübt wird.

9. Universalbohrerführungssystem (100) nach Anspruch 8, wobei der Schaft (142) des Bohreranschlags (140) Umfangsnuten (143) zum Eingriff mit der Verriegelung (126) enthält.

10. Universalbohrerführungssystem (100) nach Anspruch 8 oder 9, wobei die Verriegelung (125) einen Freigabeknopf (127) enthält, der so konfiguriert ist, dass er nach innen gedrückt wird, um die Verriegelung (126) vom Schaft (142) des Bohreranschlags (140) zu lösen.

11. Universalbohrerführungssystem (100) nach einem der Ansprüche 1 bis 10, wobei die Universalbohrerführung (110) einen ersten Satz von Markierungen (128) und einen zweiten Satz von Markierungen (148) zur Anzeige einer ausgewählten Tiefe enthält.

12. Universalbohrerführungssystem (110) nach Anspruch 11, wobei der erste Satz von Markierungen (128) eine vertikale Reihe von Markierungen (128a) auf dem Führungskörper (120) und der zweite Satz von Markierungen (148) eine vertikale Reihe von Markierungen (148a) auf dem Schaft (142) des Bohranschlags (140) enthält.

## Revendications

1. Système de dispositif de guidage de foret universel (100) comprenant :
un dispositif de guidage de foret universel (110) ;
un tube de foret (150) pouvant être inséré au moins partiellement dans le dispositif de guidage de foret universel (110) ; et
un trépan (200) pouvant être inséré dans le tube de foret (150),
dans lequel le dispositif de guidage de foret universel (110) comprend
une butée de foret (140) conçue pour venir en butée sur une surface de butée (206) du trépan (200) pour limiter une profondeur de forage du trépan (200), et
une bague de verrouillage (172) pouvant tourner entre une orientation de verrouillage, dans laquelle le tube de foret (150) inséré dans le dispositif de guidage de foret universel (110) est verrouillé par la bague de verrouillage (172), et une orientation de libération, dans laquelle le tube de foret (150) peut être libéré de la bague de verrouillage (172), **caractérisé par**
un mécanisme à effet de came (190) conçu pour faire pivoter la butée de foret (140) à distance de la surface de butée (206) du trépan (200) à mesure que la bague de verrouillage (172) est tournée dans l'orientation de libération.

2. Système de dispositif de guidage de foret universel (100) selon la revendication 1, dans lequel le mécanisme à effet de came (190) comporte une fente de came (181) formée sur la bague de verrouillage (172) et une broche suiveuse de came (145) formée sur une extrémité distale d'un arbre (142) de la butée de foret (140).

3. Système de dispositif de guidage de foret universel (100) selon la revendication 2, dans lequel la fente de came (181) et la broche suiveuse de came (145) sont conçues de telle sorte qu'une rotation de la bague de verrouillage (172) dans le sens des aiguilles d'une montre amène la butée de foret (140) à tourner simultanément dans le sens inverse des aiguilles d'une montre, faisant pivoter la butée de foret (140) à distance de la surface de butée (206) du trépan (200).

4. Système de dispositif de guidage de foret universel (100) selon l'une quelconque des revendications 1 à 3, dans lequel un ressort de compression (174) exerce une force de sollicitation sur la bague de verrouillage (172) pour solliciter la bague de verrouillage (172) dans l'orientation de verrouillage.

5. Système de dispositif de guidage de foret universel (100) selon la revendication 4, dans lequel un commutateur (173) est exploitable manuellement pour faire tourner la bague de verrouillage (172) depuis l'orientation de verrouillage jusqu'à l'orientation de libération à l'encontre de la force de sollicitation du ressort de compression (174).

6. Système de dispositif de guidage de foret universel (100) selon l'une quelconque des revendications 1 à 5, dans lequel la butée de foret (140) comprend une plaque de butée (144) s'étendant à partir d'un/de l'arbre (142) de la butée de foret (140) transversalement, la plaque de butée (144) comprenant une fente (146) ayant une dimension de fente conçue pour permettre qu'une section distale du trépan (200) passe à travers la plaque de butée (144), jusqu'à ce que la surface de butée (206) du trépan (200) vienne en butée sur la plaque de butée (144).

7. Système de dispositif de guidage de foret universel (100) selon l'une quelconque des revendications 1 à 6, dans lequel le dispositif de guidage de foret universel (110) comprend un corps de dispositif de guidage (120), et l'arbre (142) de la butée de foret (140) est conçu pour être soulevé ou abaissé par rapport au corps de dispositif de guidage (120).

8. Système de dispositif de guidage de foret universel (100) selon l'une quelconque des revendications 1 à 7, dans lequel le dispositif de guidage de foret universel (110) comprend un verrou (126) conçu pour venir en prise avec un/l'arbre (142) de la butée de foret (140) pour verrouiller l'arbre (142) par rapport au corps de dispositif de guidage (120) en exerçant une force de sollicitation.

9. Système de dispositif de guidage de foret universel (100) selon la revendication 8, dans lequel l'arbre (142) de la butée de foret (140) comporte des rainures circonférentielles (143) pour venir en prise avec le verrou (126).

10. Système de dispositif de guidage de foret universel (100) selon la revendication 8 ou 9, dans lequel le verrou (125) comporte un bouton de libération (127) conçu pour être pressé vers l'intérieur pour libérer le verrou (126) de l'arbre (142) de la butée de foret (140).

11. Système de dispositif de guidage de foret universel (100) selon l'une quelconque des revendications 1 à 10, dans lequel le dispositif de guidage de foret universel (110) comporte un premier ensemble de repères (128) et un second ensemble de repères (148) pour indiquer une profondeur sélectionnée.

12. Système de dispositif de guidage de foret universel (110) selon la revendication 11, dans lequel le premier ensemble de repères (128) comporte une série verticale de marquages (128a) sur le corps de dispositif de guidage (120) et le second ensemble de repères (148) comporte une série verticale de marquages (148a) sur l'arbre (142) de la butée de foret (140).
